# EUROPEAN PATENT SPECIFICATION

(11) **EP 0 407 693 B1**
(45) Date of publication and mention of the grant of the patent: **26.07.1995**
(21) Application number: 90106365.1
(22) Date of filing: 03.04.1990
(51) Int. Cl.: A61B 5/022

(54) **Cuff wrapping apparatus for blood pressure meter**
Umhüllungsmanchette für Blutdruckmessgerät
Manchette à enveloppe pour mesurer la tension artérielle

(30) Priority: 13.07.1989 JP 82510/89 U; 08.08.1989 JP 205261/89
(43) Date of publication of application: 16.01.1991
(73) Proprietor: OMRON CORPORATION, Kyoto 616 (JP)
(72) Inventor: Teramoto, Tsutom, c/o Omron Corporation, Nagaokakyo-city, Kyoto 617 (JP); Okada, Masamichi c/o Omron Corporation, Nagaokakyo-city, Kyoto 617 (JP); Takada, Mikio c/o Omron Corporation, Nagaokakyo-city, Kyoto 617 (JP); Oku, Akihiko, c/o Omron Corporation, Nagaokakyo-city, Kyoto 617 (JP)
(74) Representative: WILHELMS, KILIAN & PARTNER Patentanwälte

(56) References cited:
- EP-A- 0 073 123
- GB-A- 2 191 587
- US-A- 3 935 984
- PROCEEDINGS OF THE SEVENTH ANNUAL CONFERENCE OF IEEE/ENGINEERING IN MEDICINEAND BIOLOGY SOCIETY vol. 1, 27-30 September 1985, pages 500, 501, Chicago, US; G.J. LEANO et al.: "Finger Cuff Blood Pressure Monitoring Device"

## Description

The present invention relates generally to a cuff mechanism for blood pressure meters. More specifically, it relates to a finger cuff for an oscillometric sphygmomanometer.

The oscillometric method of determining blood pressure through detection of pulse oscillations was first reported by Roy and Adami in 1890. In the method, blood pressure is determined indirectly, as opposed to actually sticking a needle in the bloodstream, by occlusion of an artery by cuff pressurization. When cuff pressure is high enough to close an artery and thus stop the flow of blood, the pressure can be gradually released and compared to the blood flow returning to the artery. However, not until recently with the invention of computers and microprocessors has man been able to take advantage of this method.

The oscillometric method of determining blood pressure has traditionally involved mainly the optical oscillation method. In the optical oscillation method, a light emitting diode (LED) inside the cuff beams infrared light into the body. Since blood inside the artery absorbs more infrared than other living tissues, the amount of infrared that is exposed to a phototransistor (PTr) is a function of the blood volume in the artery. One disadvantage with this method is its reliance on light for measurement. Measurements conducted in daylight risk inaccuracy due to sunlight interfering with the infrared light. Another disadvantage with an optical oscillometric apparatus is that cuff construction for such a device is complicated and costly due to the infrared elements. Not only are the photoelectric devices expensive in and among themselves, but also they restrict bladder construction. The bladder construction must conform to the necessity of the photoelectric devices to be properly aligned with respect to the artery. Thus, bladders in prior art contain many ridges which expand together in order to tighten the cuff. Although this allows the LED and the PTr to maintain proper alignment, the complex construction is more expensive and less effective than the simple bladder employed in this invention. First, the ridges reduce the accuracy of the measured blood pressure because the measurement is a function of the bladder's surface area contact with the skin. Secondly, the need for fitting all sizes of fingers necessitates a large initial diameter into which voluminous air pressure must be pumped.

A cuff wrapping apparatus according to the preamble of claims 1 and 6 is known from US-A-3 935 984.

To overcome the parameters imposed on cuff construction by the optical oscillation method, it is a primary object of this invention to utilize a blood pressure measuring apparatus that employs a cuff oscillation method to determine blood pressure. The cuff oscillation method measures blood pressure through tiny fluctuations in the cuff pressure. Thus, the cuff pressure itself is very important

A major objective of this invention is to increase the contacting area of the cuff to the finger. Prior art involving the oscillation method was limited to this end by the necessity of having the LED and PTr properly aligned with the blood vessel. No such restrictions apply here. The cuff can fully encompass the bladder and finger, providing a snug fit.

Yet another object of this invention is to incorporate into the cuff a smooth inflatable bladder comprising a single piece of material. This simple design further helps to insure an adequate fit in which the whole surface area comes in contact with the appendage.

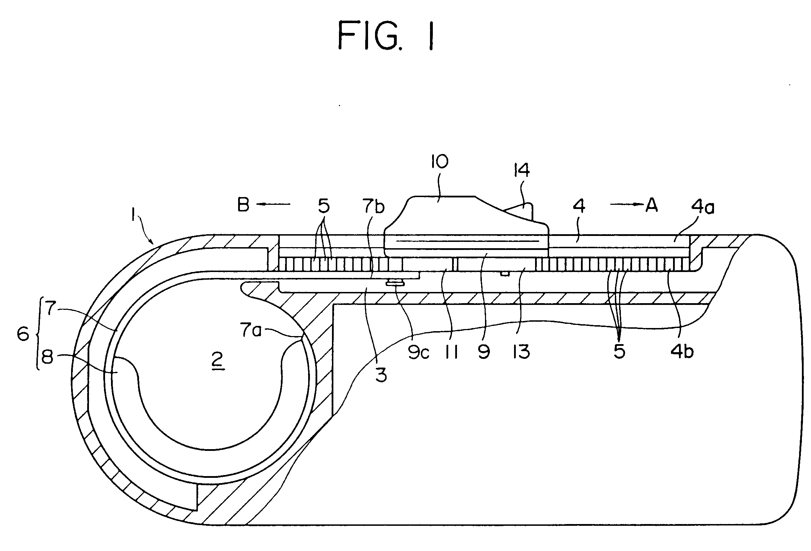

Referring to **Fig. 1**, another object of this invention is to reduce the amount of air that must be pumped into the bladder **8**. The less air needed to secure the finger in the cuff **6**, the more pronounced the changes in the cuff pressure will be during measurement and, thus, the higher the accuracy of the measurement. Therefore, the cuff **6** is initially compressed around the finger manually by pulling a cuff collar **7** down a guide track **4**. The construction of such a device is in its simplicity less expensive than the automated pumping used in the prior art and additionally provides for more accurate results in a cuff oscillation sphygmomanometer.

As, in this embodiment, the left index finger is to be cuffed, it is a further object of the invention to provide a cuff that is adaptable to fit all sizes of fingers. Thus, the initial opening **2** in the cuff **6** is large enough to accommodate any finger. The means for tightening the cuff is provided by a lever **10** attached to the end of the cuff collar **7b**. The patient himself can obtain sufficient cuff pressure by inserting his finger into an opening **2** in the cuff **6** and pushing the lever **10** down the guide track **4**. When a sufficient force has been applied to the lever **10** in a cuff winding **A** direction, a patient can begin the blood pressure measuring process.

Still another object of this invention is to provide a means of cuff tightening that is easily reproducible. Since approximately the same amount of force is applied in each simple cuff winding procedure, an operator is practically ensured of properly securing the cuff.

Furthermore, the ease and reproducibility of securing the finger in the cuff, combined with a lack of the discomfiture associated with excessive cuff pressure that was too often experienced by the patient in the past, should raise the desirability of blood pressure monitoring.

As demonstrated, a primary object of this invention is to provide a simpler and less expensive cuff. To this end, the cuff oscillation method provides a means for determining blood pressure without employing optical elements such as a light-emitting diode or phototransistor. The cuff oscillation method, in fact, simply determines arterial volume change by tiny fluctuations in cuff pressure. These fluctuations result from the rise and fall of the blood pressure during the heart cycle. The artery opens when the cuff pressure becomes lower than the blood pressure generated by the beating of the heart. However, the blood pressure, constantly changing between the systolic and diastolic pressures, rises above and falls below the gradually declining cuff pressure until the cuff pressure finally becomes lower than the diastolic blood pressure. The relationship between the opening of the blood vessel and the cuff pressure is called the volumetric change and a volumetric pulse wave can be determined. Further, this pulse wave increases for a period of time, reaches a maximum, and then decreases until the cuff pressure is finally less than the diastolic pressure. When graphed with respect to time and pressure, the amplitudes of the volumetric pulse wave visually represent what is called an envelope of oscillation. Blood pressure is determined by using this envelope curve, which represents tiny fluctuations in the cuff pressure.

The invention is as defined in claims 1 and 6.

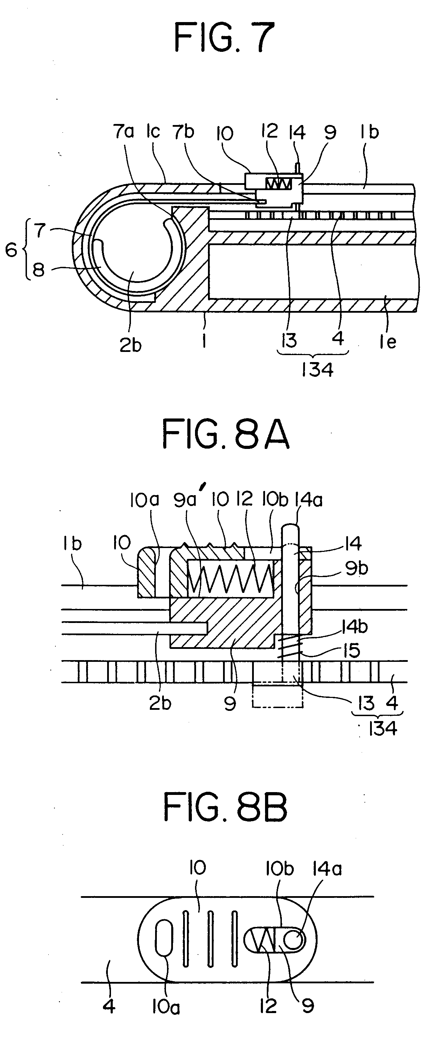

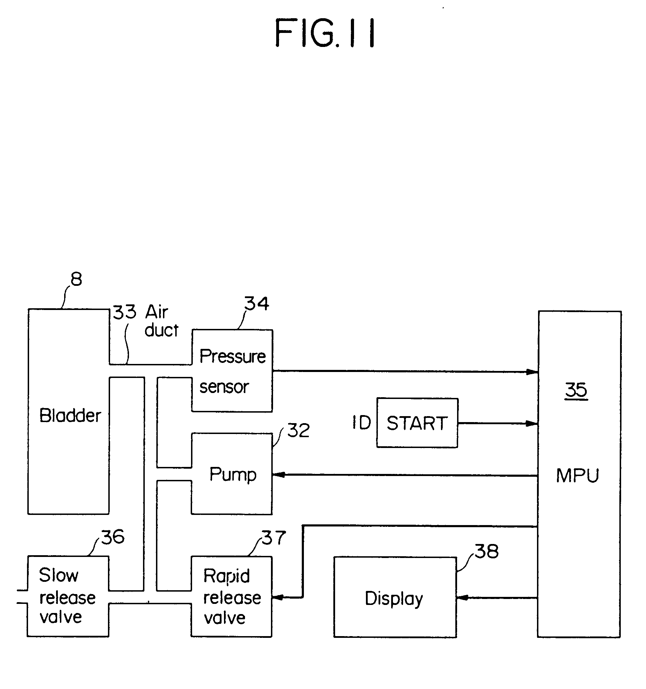

Other objects and advantages of this invention will become apparent from the following description and accompanying drawings, in which:
**Fig. (1)** is a cross-sectional view of a casing, cuff, and sliding mechanism.
**Fig. (2A)** is a cross-sectional view of the sliding mechanism before cuff pressurization operation.
**Fig. (2B)** is a cross-sectional view of the sliding mechanism after cuff pressurization operation.
**Fig. (3A)** is a bottom view of a fixing mechanism for a lever base of the sliding mechanism before cuff pressurization operation.
**Fig. (3B)** is a bottom view of the fixing mechanism for a lever base of the sliding mechanism after the cuff pressurization operation.
**Fig. (4)** is a cross-sectional view of the sliding mechanism.
**Fig. (5)** is a top view of a lever and releasing button of the sliding mechanism.
**Fig. (6A)** is a bottom view of another embodiment of the fixing mechanism for a lever base of the sliding mechanism after cuff pressurization operation.
**Fig. (6B)** is a bottom view of another embodiment of the fixing mechanism for a lever base of the sliding mechanism after cuff pressurization operation.
**Fig. (7)** is a cross-sectional view of a casing, cuff, and sliding mechanism for another embodiment of this invention
**Fig. (8A)** is a cross-sectional view of the sliding mechanism represented in **Fig. 7** before cuff pressurization operation.
**Fig. (8B)** is a top view of a knob of said slider represented in **Fig. 7** before cuff pressurization operation.
**Fig. (9A)** is a cross-sectional view of the slider represeented in **Fig. 7** after the cuff pressurization operation.
**Fig. (9B)** is a top view of the knob represented in **Fig. 7** after the cuff pressurization operation.
**Fig. (10)** is a bottom view of a fixing mechanism for the slider represented in **Fig. 7**.
**Fig. (11)** depicts a block diagram of the blood pressure meter for which the cuff wrapping appararatus was designed.
**Fig. (12)** represents a flow chart showing an operation of a blood pressure meter depicted in **Fig. 11**.
**Fig. (13)** depicts an envelope curve used to determine maximum amplitude of pulse, systolic pressure, and diastolic pressure.

Referring to **Fig. (1)**, **1** is the casing for the cuff apparatus which, in this embodiment, is made of synthetic resin. An opening **2** in the casing **1** indicates the inside of a cuff **6** and is where a finger should be placed for blood pressure measurement. The cuff **6** consists of two parts, an inflatable bladder **8** and a collar **7**. One end of the collar **7a** is fixedly attached to the casing **1** and the other end **7b** extends into a space **3** extending from cuff opening **2** in a cuff winding **A** direction and is attached to a cuff collar fixing pin **9c**, which is part of a lever base **9**. The bladder **8** is attached to the internal surface of collar **7** and encircles space **2** when the collar **7b** is extended in the **A** direction.

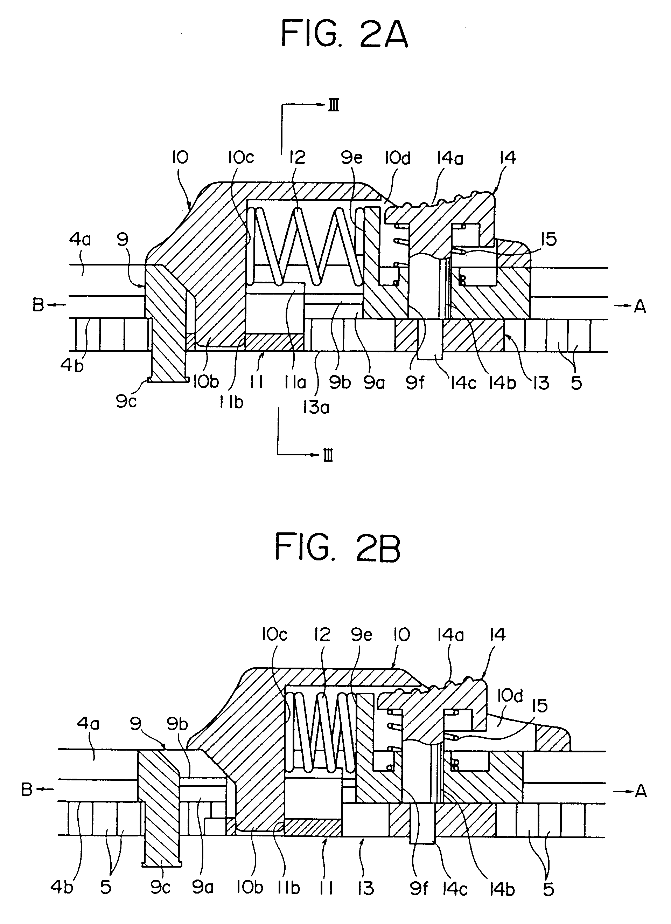

Referring to **Fig. 2a**, lever base **9**, slidable in either the cuff winding **A** direction or a cuff releasing **B** direction, is slidably attached to slide track **4** and placed on top of external step **4a**. Another part of the sliding mechanism, movable piece **11**, is also slidable in the cuff winding **A** or cuff releasing **B** direction and is mounted to slide track internal step **4b**. Additionally provided with slide track **4** are triangular teeth shaped engagement parts **5** which are used to fix the lever base **9** to the casing **1**.

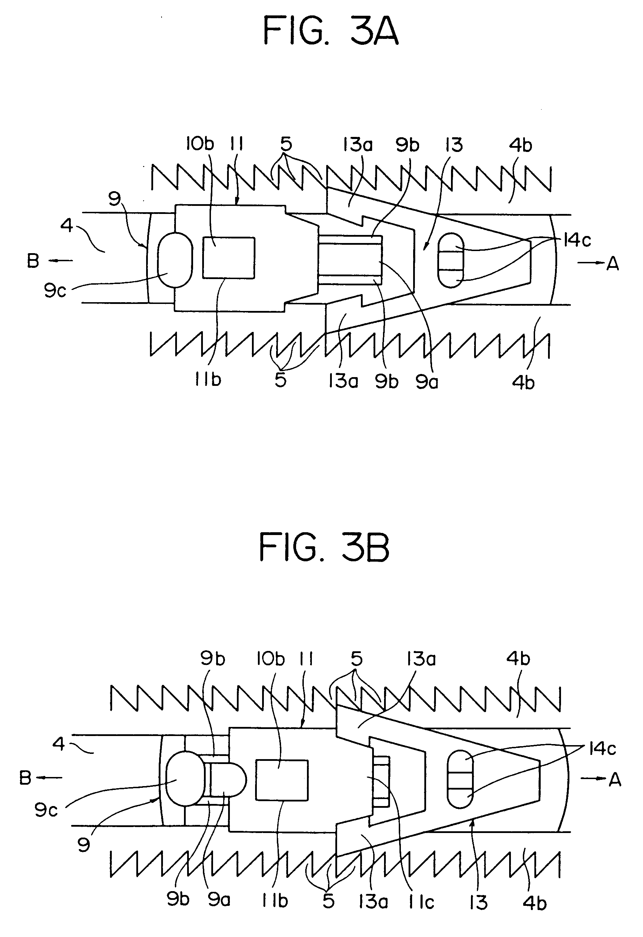

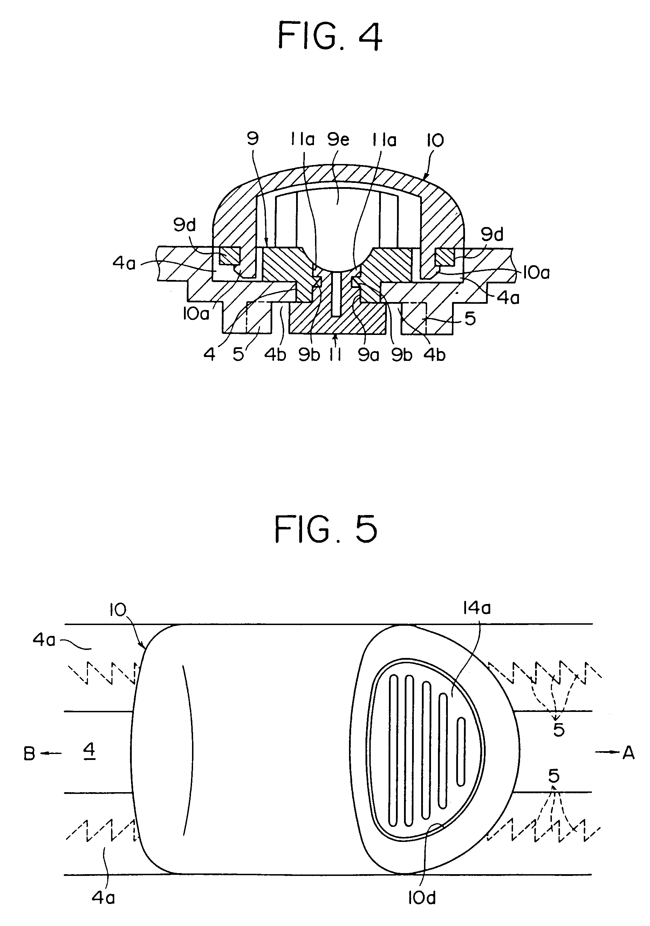

Lever base **9** is connected to a lever **10** by moving piece **11**, which is guided in the sliding direction of lever base **9** by guide track **9a**. The lever **10** projects outside of the casing **1**, enabling the patient to pull the lever base **9** down the slide track **4**, and the lever **10** also moves in the cuff winding **A** and cuff releasing **B** directions. As depicted in **Fig. 3A**, the lever **10** and the movable piece **11** are ensured of moving together by a projecting pin **10b** projecting through an inserting hole **11b** into the slide track **4**. Similarly, lever base engaging projection **9b**, formed on the inner surface of the guide track **9a**, is connected to the movable piece **11** by connecting nails **11a** which fit through the guide track **9a** and the engaging projections **9b**. **Fig. 4** illustrates the manner in which lever fixing hook **10a** is fixed to the lever base **9** by hooking the fixing part of the lever base **9d**.

Returning to **Fig. 2a**, an elastic coilspring **12**, biased in the cuff releasing **B** direction, is placed between lever base internal wall **9e** and lever internal wall **10c**. However, the embodiment is not limited to a coilspring as any elastic means will suffice. Additionally, lever opening **10d** allows head portion **14a** of a releasing button **14** to project above the casing **1**. The releasing button **14** is mounted to the lever base **9** by placing the stem portion **14b** through insertion hole **9f**. The releasing button end portion **14c** has 2 elastic portions which, as is shown in **Fig. 3A**, are inserted through a bridge shaped engaging hook **13**. Once inserted, the elastic button ends **14c** expand to effectively fix the engaging hook **13** to the button **14**. Furthermore, a coilspring **15** mounted to the release button stem portion **14b** outwardly biases the release button so that the hook **13** is normally contacting internal step **4b**. Hook tips **13a** extend from the hook **13** in order to engage with the engaging portions **5** of the slide track **4**, thus preventing the lever base **9** from sliding in the cuff releasing **B** direction. When the release button **14** is pushed, the hook **13** is downwardly released from the engaging portions **5** and, consequently, lever base **9** becomes slidable in the cuff releasing direction **B** as well as the cuff winding **A** direction.

Describing now an operation of the cuff wrapping apparatus, lever base **9** is initially located near the cuff **6**. The diameter of the cuff opening **2** is at this time large so a finger can be easily inserted into the cuff **6**. Once the left index finger is inserted, the patient can use his right hand to push the lever **10** in the cuff winding **A** direction. The force on the lever **10** is then transferred to the lever base **9** through the coilspring **12**. Consequently, the lever base **9** also travels in the cuff winding **A** direction. During this time, the engaging hook tips **13a** are deformed in such a manner, i.e. pushed inwards, so as to enable movement past the engaging portions **5** and down the slide track **4** in the cuff winding **A** direction. Thus, the cuff collar **7b** is extracted into the cuff extracting space **3** and the cuff **6** is wrapped around the finger.

Referring to **Fig. 2b**, as the cuff **6** is wound and the force applied to the lever **10** gets larger, the coilspring **12** begins to contract. According to the amount of coilspring **12** deformation, the lever **10** is displaced in the cuff winding **A** direction with respect to the lever base **9**. When the lever **10** is displaced, moving piece **11** also begins cuff winding **A** directional displacement and, as illustrated in **Fig. 3b**, a moving piece projection **11c** goes through the engaging hook tips **13a**. At the time when the most suitable winding force is applied, the projection **11c** is engaged with the engaging hook tips **13a**, consequently preventing further deformation of the tips **13a**. Thus, even if more force is exerted on the lever **10**, the hook tips **13a** can not even surpass the next engaging portion **5**. Accordingly, the lever base **9** is fixed.

Once the lever base **9** is fixed, the patient takes his hand off the lever **10**, which returns to its original position with respect to the lever base **9** due to the force from the coilspring **12**. At this time, the moving piece projection **11c** disengages from the hook tips **13a**, but the lever base **9** is still secured because the hook tips **13a** cannot move down the slide track **4** in the cuff releasing **B** direction. Finally, the inflatable bladder **8** is filled with air to compress the finger and the process of blood pressure measurement begins.

After the completion of the blood pressure measurement, the release button **14** is pushed. In turn, the engaging hook **13** drops below internal step **4b** into a hollow part of the casing **1**. Thus, the hook tips **13a** are no longer engaged with the engaging portions **5** and the lever base **9** is now freed to move in the cuff releasing **A** direction to loosen the cuff **6**. When the release button **14** is no longer pushed, coilspring **15** will return the release button to its original position. Consequently, the hook **13** will return to the internal step **4b** and the hook tips will reengage with the engaging projections **5**.

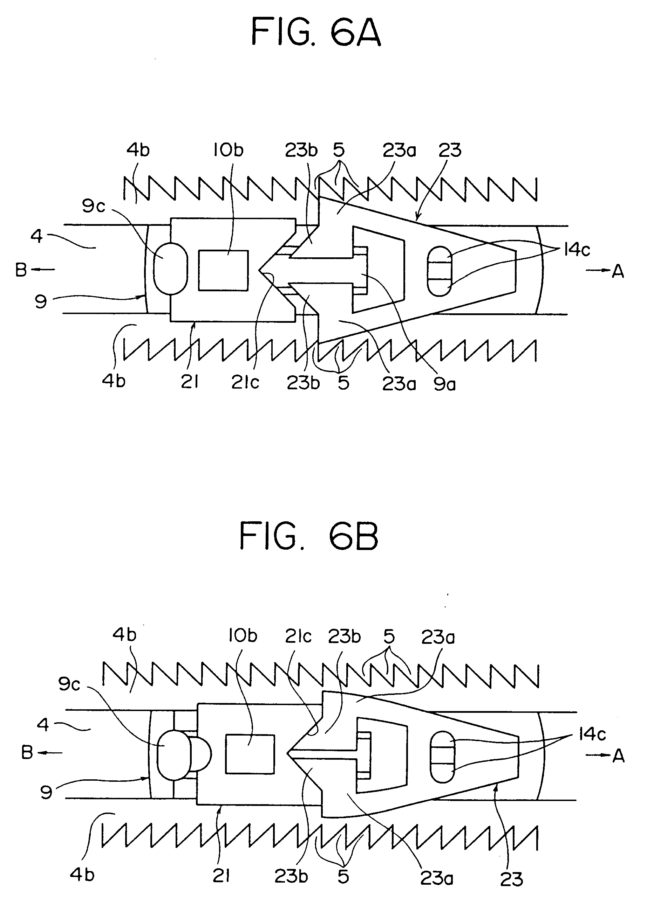

**Figs. 6a** and **6b** illustrate another embodiment of this invention. The only difference is the shape of movable piece **21** and engaging hook **23**. Recess **21c** is formed in the movable piece **21** while engaging tips **23a** have projections **23b** formed thereon. During the cuff winding process, the recess **21c** and the tip projections **23b** are apart from each other when the force applied to the lever **10** is small. Therefore, the unrestricted hook tips **23a** are able to be deformed in such a manner as to allow them to pass the engaging projections **5** in the cuff winding **A** direction. Consequently, the lever base **9** moves in the **A** direction and the cuff **6** is wound. As the force applied to the lever **10** gets larger, the movable piece recess **21c** gets closer to the tip projections **23b**. When the winding force of the cuff surpasses an appropriate value, the tip projections **23b** fit into the recess **21c** as shown in **Fig. 6b**. However, since the hook tips **23a** are no longer in contact with the engaging portions **5**, the lever base will recede a little bit in the cuff releasing **B** direction. Subsequently, the hook projections **23b** will begin to slide out of the recess **21c** until the hook tips again engage with the engaging projections **5**. At this time the lever base is fixed and blood pressure can be measured.

Yet another embodiment of this invention is depicted in **Figs. 7, 8A, 8B, 9A, 9B, and 10**. **1** is the casing for the cuff apparatus which, in this embodiment, is made of plastic. A cuff **6** is the same, consisting of the inflatable bladder **8** and the collar **7**. While one end of the collar **7a** is fixedly attached to the casing **1**, the other end **7b** extends, in this case, into a slider **9**. The bladder **8** is attached to the inside of the collar **7** and forms a circle around space **2** in order to secure the finger when the slider **4** is pulled down a slide track **1b**. Furthermore, the slide track **1b** is embedded in a top casing **1c**. In one embodiment, numbers (i.e. 1,2,3,4,5) may be printed on said top casing **1c** next to the slide track **1b** indicating cuff size.

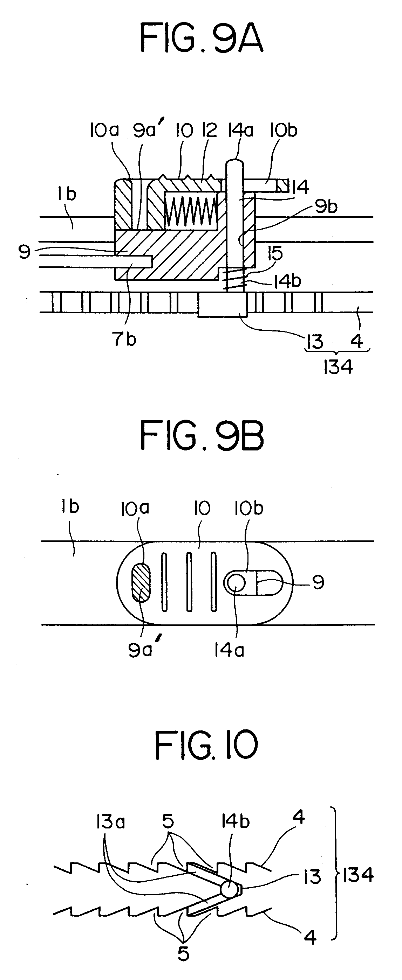

Referring to **Fig. 8A**, a knob **10** is provided above the slider **9** and protruding above the top casing **1c**. In this embodiment, a marker **9a'** is situated on top of slider **9** and a window **10a** is placed in knob **10**. As the knob **10** is pulled down the slide track **1b**, see **Fig. 9A**, the marker **9a'** can be viewed through window **10a**, see **Fig. 9B**, indicating when proper cuff pressure is attained. However, in another embodiment, the window **10a** can be eliminated and the knob **5** can be shortened to enable it to slide completely past the marker **9a'**.

Again referring to **Fig. 8A**, slider section **9** is interconnected to knob **10** by an elastic means **12**. In this embodiment, the elastic means **12** is a prestressed spring in which deformation of said elastic means **12** occurs when the cuff pressure is adequate for measuring blood pressure. Besides a spring, other elastic means may be used such as: a coil, a weight-loaded regulator, or some kind of elastic rubber like polyurethane. Means for securing the sliding mechanism is provided for in this embodiment by a pin **14** with a button **14a** on top and a coil spring **15** on the bottom section **14b** which fits into a mechanism **134** for fixing the slider . As shown in **Fig.** **8B**, the pin **14** is independent from knob **10** as a space **10b** has been provided in the knob to accommodate said pin.. Also, the slider has a small vertical space **9b** to accommodate the movement of the pin. Referring to **Fig. 10**, the fixing mechanism **134** consists of a hook **13** mounted upon the bottom of the pin **14b** and parallel to grooved track **4**. As the slider's knob **10** is pulled to secure the finger in the cuff **2**, the hook **13** correspondingly moves down the grooved track **4**. Each step of the way, hook tips **13a** lodge into teeth **5** of the track, inhibiting backward movement of the slider **9** which now is effectively secured by the pin **14**. Said grooved track **4** should be located in the middle of the casing **1** to allow room for blood pressure meter circuits stored in such casing space **1e**.

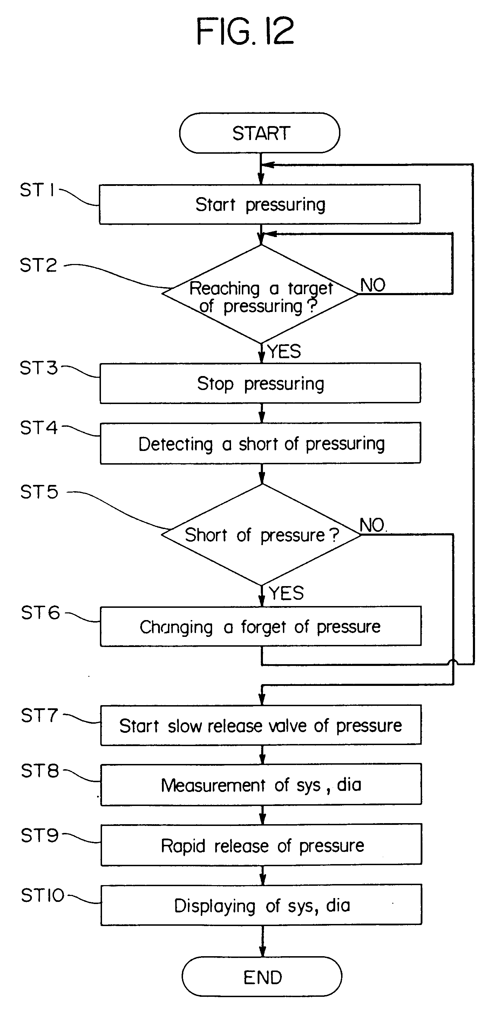

Referring to **Figs. 11 and 12**, an operation of measuring blood pressure applicable to all embodiments of this invention will be described. Once the finger has been properly secured in the bladder **8**, the patient can start measuring his blood pressure by pushing start button **1d**. When the start button **1d** is pushed, pump **12** begins to pump air through the air duct **33 (ST 1)**. At this point, a pressure sensor **34** provides data corresponding to the value of pressure in bladder **8** for a micro processing unit (MPU) **35** to judge if a target of pressure has been reached **(ST 2)**. If the MPU **35** judges no, the target pressure has not been reached, then the pump **32** will continue pumping and the pressure sensor **34** will further provide data corresponding to the increasing value of pressure in bladder **8** for the MPU **35 (ST 2)**. If the MPU **35** judges that the target pressure has been reached, then the pump **32** will stop pressuring **(ST 3)**. Next, the MPU **35** performs the function of detecting a short of pressuring in the bladder **8**; detecting whether or not the cuff pressure is sufficient for blood pressure measurement **(ST 4)**. The MPU **35** then judges whether the bladder **8** is short of pressure **(ST 5)**. If the MPU **35** judges yes there is a shortage of pressure, then it changes the target pressure **(ST 6)** and begins again at **ST 1**. If, on the other hand, no shortage of pressure is detected, then the slow release valve **36** is initiated **(ST 7)**. Incidentally, in this embodiment, the slow release valve **36** releases about 2-6 mmHg/sec. During this time, the pressure sensor **34** measures the pressure in the bladder **8** and the MPU **35** performs the function of measuring the systolic and diastolic blood pressures **(ST 8)**.

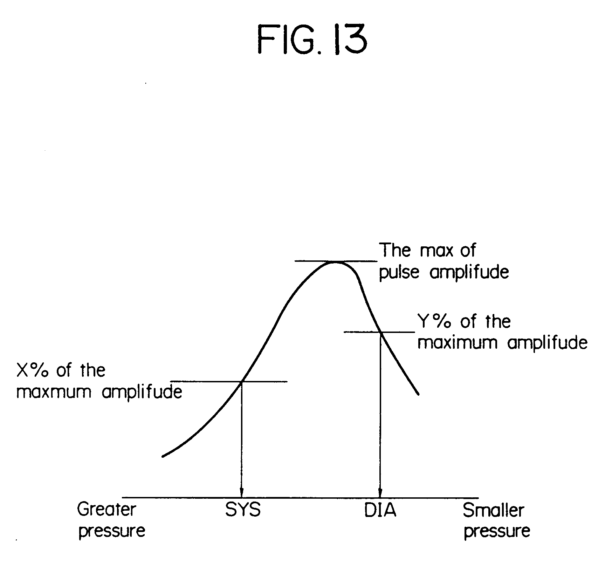

The principle of measurement can be explained by **Fig. 13**, which illustrates how the MPU **35** utilizes an envelope curve of the type described in the **SUMMARY OF THE INVENTION** to determine the systolic and diastolic blood pressures. First, the MPU **35** determines the maximum amplitude of the pulse (MAP). Secondly, the MPU **35** calculates x% of said maximum amplitude of pulse and determines the cuff pressure at which x% of the MAP occurs. This cuff pressure is the systolic blood pressure and occurs at the onset of the tiny fluctuations in the cuff pressure. Similarly, the MPU **35** then calculates y% of the MAP and the corresponding cuff pressure. This is the diastolic blood pressure and occurs at the end of the fluctuations in the cuff pressure. When the MPU **35** has measured the systolic and diastolic pressures, the rapid release valve **37** is activated to rapidly release the cuff pressure **(ST 9)**. Finally, the systolic and diastolic blood pressures are displayed on the display **38 (ST 10)**.

Now returning to a description of the third embodiment of this invention described in **Figs. 7-10**, after the blood pressures have been displayed, the patient pushes the pin button **14a** which, in turn, disengages the hook **13** from the track **4** (as shown in **Fig. 8A**), effectively releasing the slider **9** and the collar **7** to return to their original positions.

## Claims

1. A cuff wrapping apparatus for a blood pressure meter, comprising:
a) a casing (1);
b) a cuff (6) which is accomodated in a portion of said casing (1) with one end affixed thereto; characterized by:
c) a lever base (9) to which the other end of said cuff (6) is fixed and which is slidably provided with respect to said casing (1) in cuff winding and cuff releasing directions;
d) a movable lever (10) provided on said lever base (9) and which is also movable in cuff winding and cuff releasing directions;
e) an elastic body (12) which is placed between said lever base (9) and said lever (10) and which transfers to the lever base (9) force applied to the lever (10) through its own deformation;
f) a lever base fixing mechanism for fixing said lever base (9) to the casing (1); and
g) a moving piece (11, 23) which moves with the lever (10) and, when the elastic deformation of said elastic body surpasses a given level, enables said lever base fixing mechanism to enter a fixing state.

2. A cuff wrapping apparatus for a blood pressure meter as recited in claim 1, further characterized by:
said cuff (6) consisting essentially of a collar (7) with one end affixed to the casing (1), the other end linked to the lever base (9), and an inflatable bladder (8) mounted inside the collar (7).

3. A cuff wrapping apparatus for a blood pressure meter as recited in claim 1, further characterized by:
said lever base fixing mechanism consisting essentially of a release button (14), a hook (13) with hook tips (13a), a slide track (4) with projections (5) for engaging said hook tips (13a), and a movable piece (11) with a projection for securing said hook tips (13a) to said track projections (5).

4. A cuff wrapping apparatus for a blood pressure meter as recited in claim 1, further charcterized by:
said lever base fixing mechanism consisting essentially of a release button (14), a hook (23) with hook tips (23a) and hook tip projections (23b), a slide track (4) with projections (5) for engaging said hook tips (23a), and a movable piece (21) with a recess (21c) for said hook tip projections (23b) for securing said hook tips (23a) to said tack projections (5).

5. A cuff wrapping apparatus for a blood pressure meter as recited in claim 1, further characterized by:
said casing (1) comprising a hollow space beneath the lever base fixing mechanism in which circuits necessary for a blood pressure meter are located.

6. A cuff wrapping apparatus for a blood pressure meter comprising:
a) a casing (1);
b) an approximately cylindrical cuff (7) accommodated in said casing, one end of said cuff to be fixed in the casing (1); characterized by:
c) a sliding means (9) to which another end of the cuff is linked;
d) a fixing means for fixing said slider on an extending direction from said cuff (7);
e) a knob (10) provided on said slider (9) and being movable along the moving direction thereof;
f) an elastic means (12) for interconnecting said knob and slider; and
g) a marker means (9a') for indicating when the deformation of said elastic means (12) is within a certain amount of displacement.

7. A cuff wrapping apparatus for a blood pressure meter as recited in claim 6, further characterized by:
said cuff (6) consisting essentially of a collar (7) with one end affixed to the casing (1), the other end linked to the sliding means (9), and an inflatable bladder (8) mounted inside the collar (7).

8. A cuff wrapping apparatus for a blood pressure meter as recited in claim 6, further characterized by:
a marker means in which a mark (9a') is situated on top of said slider (9); and
a window (10a) provided on said knob (10) in which to view said mark.

9. A cuff wrapping apparatus for a blood pressure meter as recited in claim 6, further characterized by:
a marker means in which a mark (9a') is situated on top of said slider (9); and
the mark becoming visible when said knob (10) slides past said mark.

10. A cuff wrapping apparatus as recited in claim 6, further characterized by said fixing means comprising:
a pin (14b);
a hook (13) affixed to the bottom of said pin (14b); and
a grooved track (4) parallel to said hook (13).

11. A cuff wrapping apparatus as recited in claim 6, further characterized by:
said casing (1) comprising a hollow space located beneath the fixing means, wherein the hollow space contains circuits necessary for a blood pressure meter.

## Patentansprüche

1. Manschettenwickelvorrichtung für ein Blutdruckmeßgerät, mit
a) einem Gehäuse (1),
b) einer Manschette (6), welche in einem Abschnitt des Gehäuses (1) mit einem daran befestigten Ende aufgenommen ist, gekennzeichnet durch
c) eine Hebelbasis (9), an der das andere Ende der Manschette (6) befestigt ist und die in Bezug auf das Gehäuse (1) in Manschettenwickel- und in Manschettenlöserichtung verschiebbar vorgesehen ist,
d) einen bewegbaren Hebel (10), der auf der Hebelbasis (9) vorgesehen und auch in Manschettenwickel- und Manschettenlöserichtung bewegbar ist,
e) einen elastischen Körper (12), der zwischen der Hebelbasis (9) und dem Hebel (10) angeordnet ist und auf die Hebelbasis (9) auf den Hebel (10) ausgeübte Kraft über seine eigene Verformung überträgt,
f) einen Hebelbasisfixiermechanismus zum Fixieren der Hebelbasis (9) am Gehäuse (1), und
g) ein bewegliches Teil (11, 23), welches sich mit dem Hebel (10) bewegt und, wenn die elastische Verformung des elastischen Körpers einen gegebenen Wert übersteigt, ermöglicht, daß der Hebelbasisfixiermechanismus in einen Fixierzustand eintritt.

2. Manschettenwickelvorrichtung für ein Blutdruckmeßgerät nach Anspruch 1, ferner dadurch gekennzeichnet, daß
die Manschette (6) im wesentlichen aus einem Kragen (7) mit einem am Gehäuse (1) befestigten Ende, wobei das andere Ende mit der Hebelbasis (9) verbunden ist, und einer aufblasbaren Blase (8), die innerhalb des Kragens (7) angebracht ist, besteht.

3. Manschettenwickelvorrichtung für ein Blutdruckmeßgerät nach Anspruch 1, ferner dadurch gekennzeichnet, daß
der Hebelbasisfixiermechanismus im wesentlichen aus einem Löseknopf (14), einem Haken (13) mit Hakenspitzen (13a), einer Gleitschiene (4) mit Vorsprüngen (5) zum Erfassen der Hakenspitzen (13a) und einem beweglichen Teil (11) mit einem Vorsprung zum Festlegen der Hakenspitzen (13a) an den Schienenvorsprüngen (5) besteht.

4. Manschettenwickelvorrichtung für ein Blutdruckmeßgerät nach Anspruch 1, ferner dadurch gekennzeichnet, daß
der Hebelbasisfixiermechanismus im wesentlichen aus einem Löseknopf (14), einem Haken (23) mit Hakenspitzen (23a) und Hakenspitzenvorsprüngen (23b), einer Gleitschiene (4) mit Vorsprüngen (5) zum Erfassen der Hakenspitzen (23a) und einem beweglichen Teil (21) mit einer Ausnehmung (21c) für die Hakenspitzenvorsprünge (23b) zum Festlegen der Hakenspitzen (23a) an den Schienenvorsprüngen (5) besteht.

5. Manschettenwickelvorrichtung für ein Blutdruckmeßgerät nach Anspruch 1, ferner dadurch gekennzeichnet, daß
das Gehäuse (1) einen Hohlraum unterhalb des Hebelbasisfixiermechanismus aufweist, in welchem für ein Blutdruckmeßgerät erforderliche Schaltungen angeordnet sind.

6. Manschettenwickelvorrichtung für ein Blutdruckmeßgerät mit
a) einem Gehäuse (1),
b) einer ungefähr zylindrischen Manschette (7), die in dem Gehäuse aufgenommen ist, wobei ein Ende der Manschette im Gehäuse (1) zu befestigen ist, gekennzeichnet durch
c) Schiebemittel (9), mit welchen ein anderes Ende der Manschette verbunden ist,
d) Fixiermittel zum Fixieren des Schiebers an einer Erstreckrichtung von der Manschette (7),
e) einen Knopf (10), der auf dem Schieber (9) vorgesehen ist und längs der Bewegungsrichtung desselben beweglich ist,
f) elastische Mittel (12) zum Verbinden des Knopfes und des Schiebers, und
g) Markierungsmittel (9a') zum Angeben, wenn die Verformung der elastischen Mittel (12) innerhalb eines bestimmten Verschiebungsbetrags liegt.

7. Manschettenwickelvorrichtung für ein Blutdruckmeßgerät nach Anspruch 6, ferner dadurch gekennzeichnet, daß
die Manschette (6) im wesentlichen aus einem Kragen (7) mit einem am Gehäuse (1) befestigten Ende, wobei das andere Ende mit den Schiebemitteln (9) verbunden ist, und einer im Kragen (7) angebrachten aufblasbaren Blase (8) besteht.

8. Manschettenwickelvorrichtung für ein Blutdruckmeßgerät nach Anspruch 6, ferner gekennzeichnet durch
Markierungsmittel, bei welchen eine Markierung (9a') oben auf dem Schieber (9) angeordnet ist, und
ein an dem Knopf (10) vorgesehenes Fenster (10a), in welchem die Markierung sichtbar ist.

9. Manschettenwickelvorrichtung für ein Blutdruckmeßgerät nach Anspruch 6, ferner gekennzeichnet durch
Markierungsmittel, bei welchen eine Markierung (9a') oben auf dem Schieber (9) angeordnet ist, und dadurch, daß
die Markierung sichtbar wird, wenn sich der Knopf (10) über die Markierung hinaus bewegt.

10. Manschettenwickelvorrichtung nach Anspruch 6, ferner dadurch gekennzeichnet, daß die Fixiermittel
einen Stift (14b),
einen unten am Stift (14b) befestigten Haken (13) und
eine gerillte Schiene (4) parallel zum Haken (13) aufweisen.

11. Manschettenwickelvorrichtung nach Anspruch 6, ferner dadurch gekennzeichnet, daß
das Gehäuse (1) einen unter den Fixiermitteln angeordneten Hohlraum aufweist, wobei der Hohlraum für ein Blutdruckmeßgerät erforderliche Schaltungen enthält.

## Revendications

1. Un appareil d'enveloppement de manchette destiné à un instrument de mesure de la pression sanguine, comprenant:
a) une gaine (1);
b) une manchette (6) qui est logée dans une partie de ladite gaine (1), une de ses extrémité lui étant fixée, caractérisé par:
c) une base (9) de levier à laquelle l'autre extrémité de ladite manchette (6) est fixée et qui est disposée à coulissement par rapport à ladite gaine (1) dans les sens d'enroulement de la manchette et de libération de la manchette;
d) un levier mobile (10) disposé sur ladite base (9) du levier et qui est mobile lui aussi dans les sens d'enroulement de la manchette et de libération de la manchette;
e) un corps élastique (12) qui est placé entre ladite base (9) de levier et le levier (10) et qui transfère, par sa propre déformation, à la base (9) de levier une force appliquée au levier (10);
f) un mécanisme de fixation de la base de levier pour fixer ladite base (9) de levier à ladite gaine (1); et
g) un élément mobile (11, 23) qui est déplacé avec le levier (10) et qui permet au mécanisme de fixation de la base de levier d'entrer dans un état de fixation dès lors que la déformation élastique dudit corps élastique dépasse un niveau donné.

2. Un appareil d'enveloppement de manchette destiné à un instrument de mesure de la pression sanguine selon la revendication 1, caractérisé en outre, en ce que
ladite manchette (6) consiste essentiellement en une collerette (7) dont une extrémité est fixée à la gaine (1), et une vessie gonflable (8) montée à l'intérieur de la collerette (7).

3. Un appareil d'enveloppement de manchette destiné à un instrument de mesure de la pression sanguine selon la revendication 1, caractérisé en outre en ce que:
ledit mécanisme de fixation de la base du levier consiste essentiellement en un bouton de libération (14), un crochet (13) à pointes (13a) de crochet, une piste de coulissement (4) qui comprend des saillies (5) pour venir en prise avec lesdites pointes (13a) de crochet, et un élément mobile (11) pourvu d'une saillie pour fixer lesdites pointes (13a) de crochet auxdites saillies (5) de piste.

4. Un appareil d'enveloppement de manchette destiné à un instrument de mesure de la pression sanguine selon la revendication 1, caractérisé en outre en ce que:
ledit mécanisme de fixation de la base du levier consiste essentiellement en un bouton de libération (14), un crochet (23) pourvu de pointes (23a) de crochet et de saillies (23b) de pointes de crochet, une piste de coulissement (4) à saillies (5) pour venir en prise avec lesdites pointes (23a) de crochet, et un élément mobile (21) où est ménagé un évidement (21c) pour lesdites saillies (23b) des pointes de crochet afin de fixer lesdites pointes (23a) de crochet auxdites saillies (5) de la piste.

5. Un appareil d'enveloppement de manchette destiné à un instrument de mesure de la pression sanguine selon la revendication 1, caractérisé en outre en ce que:
ladite enveloppe (1) comprend, au-dessous du mécanisme d'application de la base de levier, un espace libre dans lequel sont disposés des circuits nécessaires pour un instrument de mesure de la pression sanguine.

6. Un appareil d'enveloppement de manchette destiné à un instrument de mesure de la pression sanguine comprenant:
a) une enveloppe (1);
b) une manchette approximativement cylindrique (7) logée dans ladite enveloppe, une extrémité de ladite manchette devant être fixée dans l'enveloppe (1), caractérisé par:
c) un moyen coulissant (9) auquel une autre extrémité de la manchette est reliée;
d) un moyen de fixation pour fixer ledit coulisseau sur une direction qui s'étend à partir de ladite manchette (7);
e) un bouton (10) disposé sur ledit coulisseau (9) et mobile le long de ladite direction de déplacement de celui-ci;
f) un moyen élastique (12) pour relier entre eux ledit bouton et ledit coulisseau; et
g) un moyen marqueur (9a') pour indiquer quand la déformation dudit élément élastique se trouve à l'intérieur d'une certaine amplitude de déplacement.

7. Un appareil d'enveloppement de manchette destiné à un instrument de mesure de la pression sanguine selon la revendication 6, caractérisé en outre en ce que:
ladite manchette (6) consiste essentiellement en une collerette (7) dont une extrémité est fixée à la gaine (1) et dont l'autre extrémité est fixée au moyen coulissant (9), et une vessie gonflable (8) montée à l'intérieur de la collerette (7).

8. Appareil d'enveloppement de manchette destiné à un instrument de mesure de la pression sanguine selon la revendication 6, caractérisé en outre par:
un moyen marqueur dans lequel une marque (9a') est située sur le sommet dudit coulisseau (9); et
une fenêtre (10a), disposée sur ledit bouton (10), dans laquelle ladite marque peut être vue.

9. Un appareil d'enveloppement de manchette destiné à un instrument de mesure de la pression sanguine selon la revendication 6, caractérisé en outre par:
un moyen marqueur dans lequel une marque (9a') est située au sommet dudit coulisseau (9); et
la marque devient visible lorsque ledit bouton (10) coulisse au-delà de ladite marque.

10. Un appareil d'enveloppement de manchette selon la revendication 6, caractérisé en outre en ce que ledit moyen de fixation comprend:
une broche (14b);
un crochet (13) fixé sur le bas de ladite broche (14b); et
une piste rainurée (4) parallèle audit crochet.

11. Un appareil d'enveloppement de manchette selon la revendication 6, caractérisé en outre en ce que
ladite enveloppe (1) comprend un espace creux situé au-dessous du moyen de fixation, espace creux qui contient des circuits nécessaires pour un instrument de mesure de la pression sanguine.
